# EUROPEAN PATENT SPECIFICATION

(11) **EP 2 004 292 B1**
(45) Date of publication and mention of the grant of the patent: **11.04.2012**
(21) Application number: 07711264.7
(22) Date of filing: 13.03.2007
(51) Int. Cl.: A61N 5/06, A61M 21/00

(54) **A COMPUTER CONTROLLED LIGHT THERAPY APPARATUS**
COMPUTERGESTEUERTES LICHTTHERAPIEGERÄT
APPAREIL DE PHOTOTHÉRAPIE COMMANDÉ PAR ORDINATEUR

(30) Priority: 14.03.2006 DK 200600357
(43) Date of publication of application: 24.12.2008
(73) Proprietor: Andersen, Soren Ree, 4600 Koge (DK)
(72) Inventor: Andersen, Soren Ree, 4600 Koge (DK)
(74) Representative: Zacco Denmark A/S
(86) International application number: PCT/DK2007/000121
(87) International publication number: WO 2007/104309

(56) References cited:
- EP-A- 1 386 638
- WO-A-2005/025470
- US-A1- 2001 008 973
- US-A1- 2003 187 486
- US-A1- 2004 095 746
- US-A1- 2006 009 822
- US-B1- 6 488 698

## Description

The present invention relates generally to light therapy apparatuses. In particular, the present invention relates to a computer controlled apparatus for subjecting a person to ocular light illumination for improvement of the person's well being.

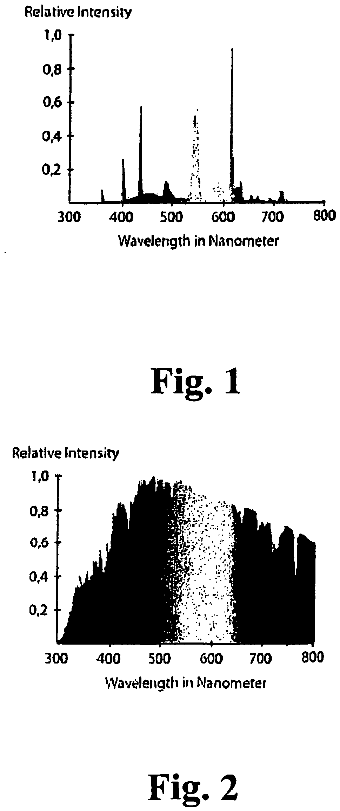

It is well known that individuals working indoor suffer from insufficient daylight illumination, and it has been estimated that millions of people suffer from so-called "light starvation". The difference between indoor light and natural daylight is illustrated in Figs. 1 and 2. Fig. 1 shows the emission spectrum of fluorescent tubes, and Fig. 2 shows the spectrum of sunlight. The differences are apparent.

The positive influence of daylight on the human brain has been known for thousands of years. Light therapy or photo therapy are the medical expressions when patients are exposed to light with a spectrum similar to daylight. Light therapy has been recognized and used for more than 20 years and many severe psychiatric disorders, such as various types of depressions, are responding to daylight stimulation.

The function of the human body and brain is controlled by various hormones, for example serotonin and melatonin. They are both important to the well being of a person and their functions are closely related. Lack of serotonin may lead to psychiatric disorders, such as polar, bi-polar and post partum depression, etc., that respond to daylight stimulation. The hormone melatonin influences the human circadian rhythm.

Like serotonin, melatonin is also related to different types of depressions. Seasonal affective disorder, also known as winter depression, is one example of how the human organism is responding to an imbalance of the two hormones.

Lack of energy and low spirits are some of the common symptoms of "light starvation", and more severe symptoms also occur, such as general depression, sleep disorders and shift-work disorders, post- and ante-partum depression, etc. It is well known that the general health and well being and also disorders of the above-mentioned type and other disorders may be treated with ocular light therapy.

Conventionally, light therapy systems have included powerful light sources emitting light of a high intensity and with a light spectrum similar to the natural daylight spectrum. Typically, fluorescent lamps have been used for this purpose emitting high intensities of light. Thus, many commercial light therapy units have been large, bulky and cumbersome.

In the last decade, advances in ballast and fluorescent light technology have allowed some companies to produce smaller, lighter-weight ocular light therapy units. An example is disclosed in US 6,488,698. Such units, though smaller and less cumbersome than previously known units, are usually too large to be hand-held. Further, the disclosed device has no display or other means to convey information.

Recently, light therapy apparatuses utilizing light emitting diodes (LEDs) have emerged. US 2006/0009822 discloses a light therapy apparatus delivering ocular light to a person to treat a disorder that is responsive to ocular light therapy. The apparatus comprises a power supply, a hand-held light output device having light sources powered by the power supply, and a programmable data processor coupled to the power supply and the light output device. In some embodiments, the plurality of light sources may provide a light output having at least forty percent blue light with a wavelength range of approximately 435 nm to 500 nm. In some embodiments, the programmable data processor is configured to control light emissions in accordance with user-defined light therapy programs.

An illumination apparatus configured to generate color light in a pleasing fashion according to a variety of visual modes is disclosed in US 2004/0095746. The illumination apparatus disclosed therein may also be configured to generate light with frequency components suitable for relieving light deficiency disorders such as reasonal affective disorders, depression, circadian rhythm disorders or the like. According to an aspect of the apparatus, the illumination device may be mounted to a computer monitor.

Conventional light therapy apparatuses with LED light sources tend to be harsh to the eyes and create retinal after imaging. Moreover, prior art LED devices are of limited portability because of power consumption that requires access to an external power outlet or relatively large cumbersome batteries, rather than using a portable or built-in battery pack. Further, known light therapy apparatuses are adapted to emit high intensity light from a position immediately in front of the user. This combination of high emitted light intensity and positioning of the light source makes it inconvenient if not impossible for the user to perform deskwork, such as work with a computer, when using the apparatus.

Thus, it is an object of the present invention to provide a convenient light therapy apparatus that allows a user to perform deskwork, in particular use of a personal computer, during illumination by the apparatus.

According to the present invention, the above-mentioned and other objects are fulfilled by provision of a light therapy apparatus comprising a computer, a light emitting assembly holding a plurality of light emitting diodes for emission of light and having a computer interface, such as a USB interface, for connection with the computer for control and power supply of the light emitting assembly and a presence detector for detection of a person present in the field of emission of the light emitting assembly, wherein the computer is adapted to control emitted light intensity to be appropriate for light therapy and whereby the user may simultaneously perform computer work during treatment, and wherein the time that a person receives light treatment as detected by the presence detector is recorded.

Provision of a computer interface for power supply of the light emitting assembly eliminates the need for a power supply in the light emitting assembly thereby decreasing the size and weight and cost of the light emitting assembly.

Further, the light emitting assembly is computer controlled through the computer interface. For example, the computer may set the emitted light intensity. This eliminates the need for a computer in the light emitting assembly further decreasing the size and weight and cost of the light emitting assembly.

The computer interface may be a USB interface, which presently forms a part of every personal computer making power supply for and control of the light therapy apparatus according to the present invention readily available for the intended users.

Preferably, the user controls the functioning of the light emitting assembly utilizing the user interface of the computer, e.g. the user may adjust various parameters, such as exposure time, shape of emitted light spectrum, the intensity of the emitted light, etc., with the computer user interface.

The computer may further be adapted to execute different light exposure programs for varying intensity and possibly spectral shape as a function of time. Such programs may be downloaded and uploaded via a network, such as the Internet. The computer may further be adapted for user programming of light exposure programs.

The light emitting diodes of the light emitting assembly may be of a specific colour or multicoloured diodes or white light emitting diodes. The light emitting assembly may also have a white light source of another type than a light emitting diode. In one embodiment, the light emitting assembly has light diodes of the same colour, and in another embodiment the light emitting assembly has light diodes of different colours. By selection of light diodes emitting different light spectra, the light emitting assembly may be designed to emit a specific light spectrum suitable for relieving a specific disorder of a specific user.

Further, the computer and the light emitting assembly may be adapted for individual control of the emitted light intensity of each type of light source or of different wavelengths of the light spectrum so that the computer may adjust the shape of the light spectrum emitted by the light emitting assembly. The shape of the spectrum may be varied as a function of time.

Typically, indoor working people will experience an increased well being and hence higher productivity upon exposure to light emitted by the light emitting assembly, since the light emitting assembly compensates for the spectral differences between natural daylight and indoor lighting. Recently, it has also been found that the effects of anti-depressive medication can be promoted and maintained by a combination of medication and light therapy.

It is an important advantage of the present invention that the light emitting assemblies may be positioned at an angle away from the field of view of the user so that light is emitted with an intensity and directionality that makes it possible for the user to perform computer work without being bothered by the light emitted from the light therapy apparatus according to the invention. This means that the treatment may have a long duration, e.g. several hours, as opposed to known light therapy apparatuses that emit light of a high intensity for a limited time period, e.g. half an hour or one hour. Thus, the light treatment with the inventive apparatus simulates exposure to natural daylight better than known apparatuses, e.g. the user may be subjected to light treatment during his or her working day, i.e. for app. 8 hours. This makes it possible to combine treatments of very long durations with the daily activities that the user has to perform.

Further, the intensity and directionality of the emitted light leave other persons in the room unaffected.

In a preferred embodiment of the invention, the light therapy apparatus comprises a light emitting assembly holding a plurality of light emitting diodes (LEDs) for emission of blue light having a wavelength in the range from 435 nm to 500 nm, and preferably the assembly emits blue light with a wavelength of 464 nm, which is an optimum wavelength for inhibiting production of melatonin.

Typically, artificial electrical light sources emit light with very little energy in the wavelength range from 435 nm to 500 nm, and especially at 464 nm, as compared to natural daylight. Therefore, many persons working indoor experience a lack of exposure to these wavelengths and suffer from "light starvation" with one or more of the above-mentioned symptoms.

Melatonin is a hormone that has sleep-inducing properties and regulates a balanced physiological state in humans. The production and suppression of melatonin is a circadian driven event. Melatonin is produced by the pineal gland in darkness and is suppressed by the interruption of darkness. The synthesis and suppression has been shown to be particularly sensitive to presence of blue light even at low intensities of blue light. The balance of melatonin and other light sensitive hormones is known to be of significant importance to a person's general health and well being, and advantageously the light therapy apparatus according to the invention is used for preventive light therapy compensating for the lack of natural daylight exposure to the blue wavelengths thereby improving the general health and well being of the user.

In a preferred embodiment, the light therapy apparatus comprises two assemblies, each of which holds a plurality of light emitting diodes for emission of light, for example blue light having a wavelength in the range from 435 nm to 500 nm, and a computer interface for connection with a computer for power supply of the assemblies. Preferably, the assemblies are positioned symmetrically on opposite sides of the intended line of sight of the user during operation, for example with a computer positioned within the line of sight of the user between the two assemblies so that the user may perform computer work during light treatment.

As used herein, the term "blue light" refers to light having wavelengths in the range of approximately 435-500 nanometres (nm). A wavelength of approximately 464 nm has been found optimum for suppression of melatonin production.

Other wavelengths may be used in a device according to the present invention in order to specifically modulate or increase endogenous serotonin production.

The light emitting assembly is preferably a portable assembly, and preferably the light emitting assembly has a light emitting surface that is less than 400 cm², such as less than 300 cm², e.g. less than 200 cm², etc. For example, the height of the light emitting assembly may be less than 30 cm and the width may be less than 5 cm and the depth may be less than 2 cm. In a preferred embodiment, the height of the light emitting assembly matches the height of the computer monitor.

Preferably, the light emitting assembly including the connections and wiring for attachments to a computer weighs less than 1 kg, such as less than 800 g, preferably less than 600 g, more preferred less than 400 g, and even more preferred less than 200 g.

The light therapy apparatus further comprises a conventional presence detector for detection of a person present in the field of emission of the light emitting assembly. The light therapy apparatus may further be adapted to automatically emit light when a person is present and to automatically stop light emission when the person leaves. This minimizes the energy consumption. Further, the time that the person receives light treatment as detected by the presence detector may be recorded.

The computer may for example perform the presence detection by detection of user activity, e.g. by detection of movement of the mouse, use of the keyboard, etc., as is well known in the art. The presence detector may comprise a video camera, such as a web camera, an IR-detector, or another presence detector as is well known, e.g. in the art of alarm systems.

Thus, according to a preferred use of the claimed invention, a method of light therapy is provided wherein light is emitted towards a person for preventive light treatment of the general health and well being of the person, and optionally to treat a disorder that is responsive to ocular light therapy. The method comprises emission of light to the eyes of the person.

The computer controlling the one or more assemblies may set the emitted light intensity in response to entry of a user command and/or the computer may adjust the emitted light intensity as a function of time in response to entry of a user command.

For example, the emitted light intensity may be adjusted as a function of time corresponding to the daylight as a function of time at a selectable position on the earth.

The light emitting assemblies may be integrated in the computer display, e.g. with light emitting assemblies positioned adjacent opposite vertical sides of the display screen.

The above and other features and advantages of the present invention will become more apparent to those of ordinary skill in the art by describing in detail exemplary embodiments thereof with reference to the attached drawings in which:
Fig. 1 shows the light spectrum emitted by fluorescent tubes,
Fig. 2 shows the spectrum of sunlight,
Fig. 3 is a photo of a light therapy apparatus with two assemblies connected to a portable computer for control and power supply,
Fig. 4 schematically illustrates an embodiment of the invention,
Fig. 5 schematically illustrates the use of an embodiment of the invention,
Fig. 6 shows a side view of a portable, light emitting assembly,
Fig. 7 shows a user interface tool bar according to the invention,
Fig. 8 shows a user programming interface according to the invention, and
Fig. 9 shows a display window for user selection of parameters of a light therapy programme.

The present invention will now be described more fully hereinafter with reference to the accompanying drawings, in which exemplary embodiments of the invention are shown. The invention may, however, be embodied in different forms and should not be construed as limited to the embodiments set forth herein. Rather, these embodiments are provided so that this disclosure will be thorough and complete, and will fully convey the scope of the invention to those skilled in the art. Like reference numerals refer to like elements throughout.

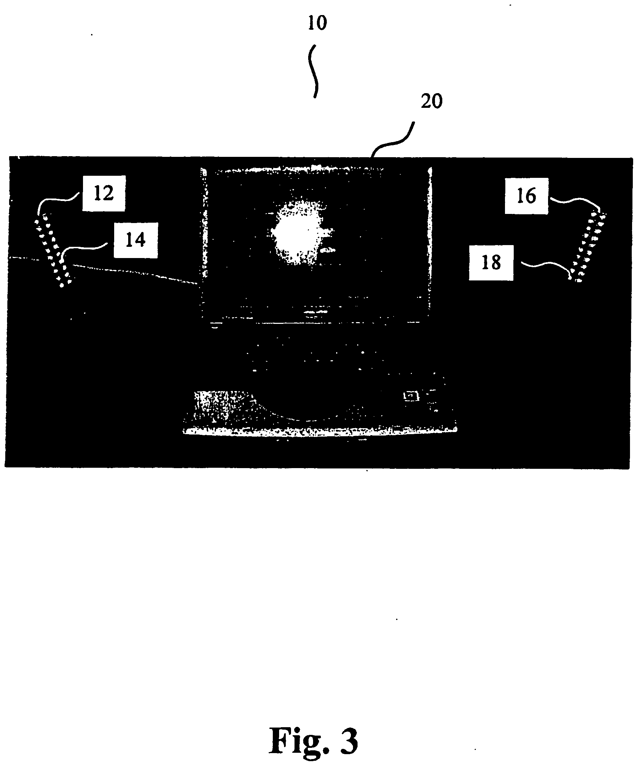

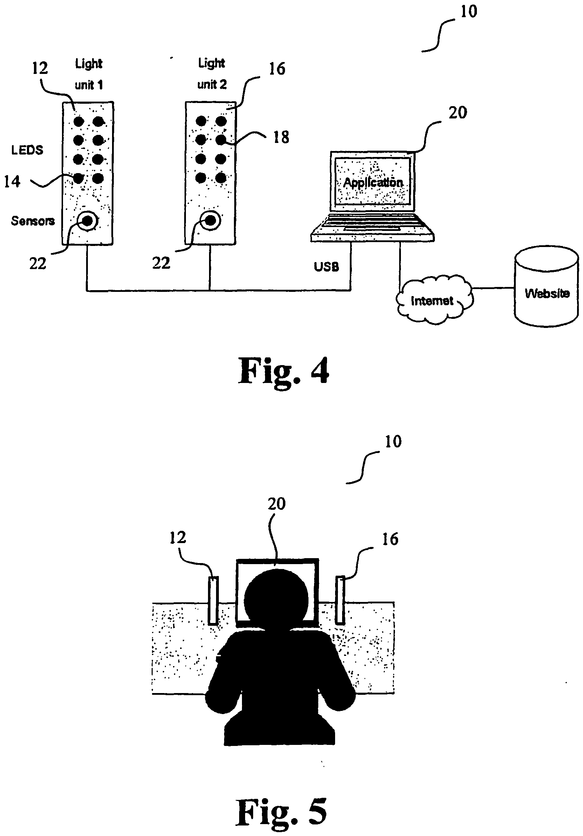

Figs. 3-5 show a front view of a preferred embodiment of the light therapy apparatus 10 comprising a first light emitting assembly 12 positioned on a table to the left of the intended line of sight of the user and holding a plurality of LEDs 14 for emission of light, for example blue light having a wavelength in the range from 435 nm to 500 nm, and a similar second assembly 16 positioned to the right of the intended line of sight of the user and holding a plurality of LEDs 18. The first 12 and second 16 light emitting assembly is connected to a USB port (not visible) of a computer 20 positioned within the intended field of view of the user between the first 12 and second 16 assembly. The computer 20 supplies power to the assemblies 12, 16 through the USB port and runs a programme that controls the intensity and possibly the spectral shape of the light emitted by the assemblies 12, 16 whereby the user may simultaneously perfom computer work during treatment with the light therapy apparatus 10.

The computer 20 may comprise different customizable light therapy treatment programs for use by different individuals and/or for different types of treatment. The computer 20 may control and record the starting day and ending day of a treatment, the time of day and duration of each treatment and accumulated in accordance with timing parameters detected by the presence detector. A number of treatments may be performed on the same day.

The light emitted by the light emitting assembly according to the present invention has been found to provide less glare, intensity and harshness to the user's eyes. Further, the emitted blue light is very effective in provision of an appropriate balance of melatonin and other light sensitive hormones.

The light therapy apparatus 10 according to the invention has proven effective for preventive light therapy and for treatment of light-related problems, such as circadian rhythm problems, seasonal affective disorders, some forms of depression, sleep disorders, and shift-work disorders, jet lag, post-partum and ante-partum depression, pre-menstrual syndrome, late luteal phase dysphonic disorder (LLPDD), bulimia and eating disorders, and chronic fatigue.

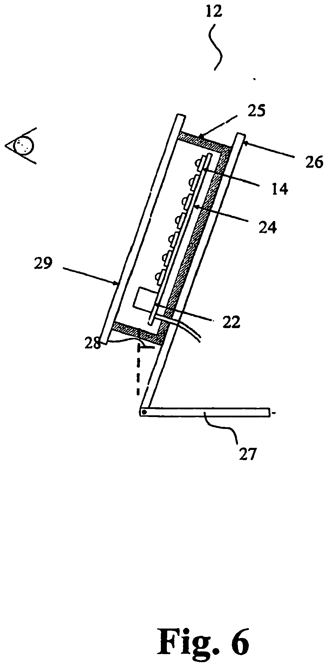

Fig. 6 is a side view of one of the portable light emitting assemblies 12, 16, comprising an array of LEDs 14, 18 with two columns of LEDs 14, 18 each. The assembly also has an infrared presence detector 22. The diodes 14, 18 and the presence detector 22 are mounted on a printed circuit board 24 mounted in a housing 25 attached to a frame 26 having a tiltable base member 27 for positioning on a horizontal support surface, such as a table. Alternatively, the frame 26 is adapted for positioning on the side of the computer monitor, for example in a way well-known from the art of loudspeakers, possibly on an arm pivotally connected to the computer monitor or extendable from the computer monitor.

In an operational position, the printed circuit board 24 forms an angle 28 of approximately 25° with respect to a vertical axis so that the surface of the printed circuit board 24 is approximately perpendicular to a line connecting the LED array with an eye of the user whereby a significant part of the light emitted by the LED array is directed towards the eye of the user.

The LED array is covered by a diffusing surface 29 for emission of diffused light towards the user to soften the light intensity and providing a more uniform field of treatment for user comfortable illumination.

The LEDs may be five millimetre LEDs.

The intensity of light emitted by the assembly 12, 16 may be less than 10000 lux, such as less than 8000 lux, such as less than 6000 lux, such as less than 4000 lux, such as less than 2000 lux at a distance ranging from approximately 15 cm to 100 cm, such as from 15 cm to 50 cm, such as from 15 cm to 30 cm, from the LED array.

Blue light has been found to be particularly effective for certain applications such as adjustment of "jet-lag" and other modulations of the circadian rhythm, when the light has a peak wavelength within the range of approximately 435 nm to 500 nm. Light sources that are configured to emit concentrated blue-coloured light have been found to provide excellent melatonin balance improving the general health and well being of the user.

Blue-coloured light LEDs have been found to be effective at lower power levels and/or greater distances than full spectrum light. For example, blue light therapy may provide useful treatment at distances of 20 to 75 cm between the person and the light source, with an especially effective distance range being about 50 to 65 cm. In one embodiment, at a distance of 50 cm, effective blue light therapy is provided at only about 400 lux, or about 2.4x 10-4 watts/cm².

The illustrated light emitting assemblies are very small and light and indeed portable. The assembly shown weighs less than 200 g and has a height of 10 cm, a width of 3 cm and a depth of 1 cm.

The computer 20 may display the time of day, data being entered into the computer, the status of the light therapy apparatus 10, the light therapy program being active, various light therapy programs that may be selected, the elapsed time of treatment, and the time remaining for the current light therapy treatment, the accumulated light received during treatment over a selected period of time, etc.

Data may be entered by a user to provide data and/or parameters to the computer in order to vary the timing or intensity of light emissions, or to set up one or more personalized light therapy programs that may be actuated at will or by a computer clock.

Thus, for example, the computer may include software for variation of the intensity of the emitted light as a function of time, e.g. similar to the variation of the intensity of natural daylight as a function of time during the day. The computer keyboard or mouse may be used to provide plus (increase) and minus (decrease) functions, such as to adjust the amount of light that the light source provides by changing the intensity of the light source. By pushing a button designated as plus, the intensity will be increased so that the light source is brighter. A button designated as minus causes the intensity of the light source to decrease. In this way, the user may adjust the emitted light intensity to a level that is comfortable for that user. The buttons referred to in the description may be physically present on the device or they may be displayed on the computer monitor as part of the user interface of the program controlling the light emitting apparatus, so they can be controlled by the user using the mouse or other control options used for interactions with the computer.

In the same way, the computer clock may be used for timing control. A user may select the amount of time in minutes that the assembly is to be actuated, thereby eliminating any need to watch the clock. The time will count down and automatically turn off the light source when the designated time has elapsed. The time may be displayed on the computer display.

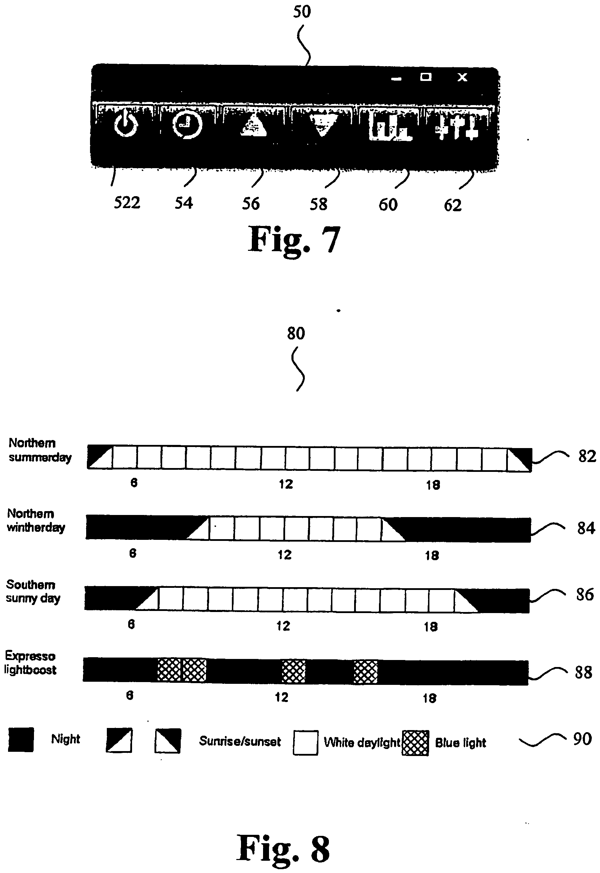

Fig. 7 shows a tool bar 50 with various tool icons displayed by the computer for user control of the light therapy apparatus.

The switch 52 is used to click light emission on and off and possibly toggle through various modes, for example presence detector on and off, white light on and off, blue light on and off, etc.

A click on the program tool 54 opens a program list window of various programs, for example, "gentle wake-up", "sunny day at the Mediterranean", "blue light espresso", "northern winter day", "northern summer day", "daylight on location and time", "time zone travel", "shift work", "user operated instrument", "phase advance treatment, "phase delay treatment", "adjunctive treatment to Citalopram medication", "dawn and dusk simulation", etc.

The tool 56 is used to increase the intensity of the emitted light, and the tool 58 is used to decrease the intensity of the emitted light.

The tool 60 is used to open a statistics window with for example a progress bar showing elapsed time and level of light exposure, accumulated weekly exposure, accumulated monthly exposure, sun diagram, etc.

The tool 62 is used to open a setting menu for adjustment of the various parameters of the light therapy apparatus. One button in the menu opens a graphical light programme editor 80 as shown in Fig. 8 in which the user may define a user light therapy programme 82, 84, 86, 88 as illustrated by four examples in Fig. 8 by drawing and connecting various tools 90 shown at the bottom of Fig. 8.

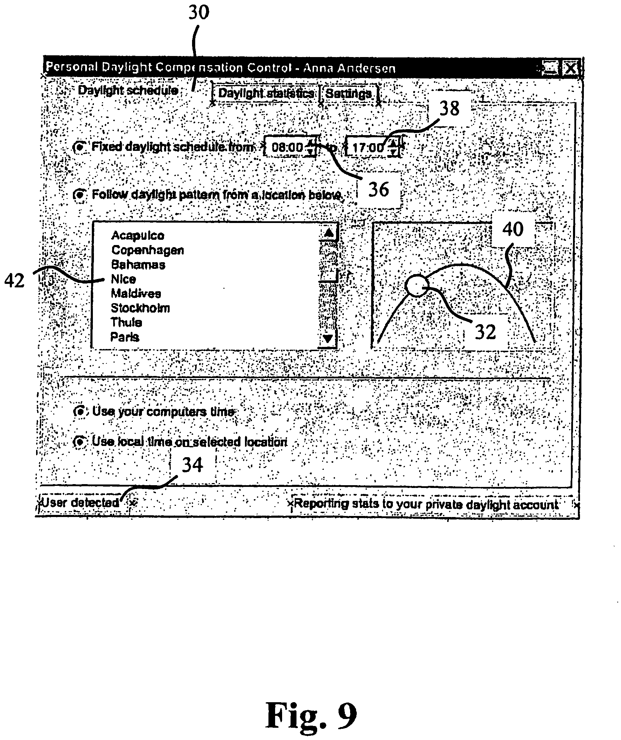

Fig. 9 illustrates a display window 30 displayed by the computer 20 that shows various information, including but not limited to current time, timer, current light intensity 32, current program 34 and battery power remaining. The computer 20 may also control a built-in alarm device or alarm clock (not shown) to alert the user at various times, such as a wake-up time or a time for therapy treatment to begin or end. The computer keyboard or mouse may be used to adjust and modify the operation of the alarm device. The keyboard or mouse may also access a built-in calendar to arrange for multiple light therapy sessions on selected days.

In this way, data and parameters may be provided to the computer in order to set up one or more light therapy programs that may be actuated at will or automatically in accordance with the internal computer clock and calendar.

For example, a user may input a desired start time 36 and stop time 38 of light therapy together with a desired light intensity for the light therapy. The emitted light intensity may be programmed to vary as a function 40 of time during therapy. For example, the light intensity may vary as a function of time like the intensity of natural daylight as a function of time at a selected location 42 on the earth. Further, the user may assign the program a name, number, or other identifier and store the data and parameters in the memory under that name. Alternatively, or additionally the light therapy programme and/or the data and parameters may be stored on a website so that the user can access the programme and/or the data and parameters, e.g. with a user key and an access code, and download desired data and parameters to setup a desired light therapy program in any computer with network access, such as internet access, and connected to at least one light emitting assembly according to the present invention to subject the user to the desired light treatment. In this way, the user can have the same information available at various locations around the world making it possible for him or her to use their own customized light therapy programmes in any location with network access and also consolidate statistical information from such locations.

The statistics relating to the duration of each treatment may be send to the users private daylight register or account on the internet for accumulation providing long term statistic information for the user.

Thus, when a user is ready to use the light therapy apparatus 10 for his/her therapy, he or she enters the pre-assigned identifier of the light therapy program and data and parameters that may reside in the computer, or may be downloaded through a network, such as the internet. The light therapy program may then be actuated as desired. Any of the stored sets of light therapy data and parameters may be recalled or downloaded and actuated. The programs may be set up to be automatically actuated at designated times by the computer clock. The built-in calendar may also be used to trigger operations of the light therapy program over several days, weeks, months or even years, as desired.

The computer may include a jet-lag calculator to be used by travellers to change sleep patterns and circadian rhythms during travel. The computer clock may monitor time across time zones and display the time at the current location. The jet-lag calculator may advice a user during travel when to use the apparatus 10 and the amount of light usage. The data may also advise the user when to avoid outdoor light.

Examples of data that may be input to the computer can include the departure airport, arrival airport, natural sleep time and natural wake time. It is known that, in order to achieve the best adjustments in the circadian rhythm, light should be administered relative to the time when the core body temperature is at a minimum. It is also known that, typically, the core body temperature minimum occurs about two hours before the natural wake up time.

The time to expose a person to light also depends on whether the person is travelling eastbound or westbound. If the person is eastbound, the circadian rhythm adjustment is best made if light is administered after the time when the core body temperature is at a minimum. If the person is westbound, the circadian rhythm adjustment is best made if light is administered before the time when the core body temperature is at a minimum.

Accordingly, data entered into the computer may include the number of time zones travelled, the direction of travel, and the core-body temperature of the traveller. The process then determines whether the traveller is headed east or west. The process then uses westbound sleep and light schedules or eastbound sleep and light schedules to calculate a sleep/wake, light/dark regimen and Instructions to facilitate the avoidance of jet lag problems. The computer display may provide function and text displays to provide the results of the jet-lag calculations.

The data input regarding a person might also include data regarding whether the person is "sleep delayed" or "sleep advanced." A sleep delayed person tends to stay up later and have a more difficult time awakening in the morning, whereas a sleep advanced person tends to want to go to bed earlier and get up earlier. This data could require separate westbound and eastbound schedules, depending whether the person was sleep delayed or sleep advanced.

In one implementation of a light therapy method, the user input is the natural wake up time and the natural fall-asleep time. From this data, the computer may calculate the time at which the core body temperature is expected to be at a minimum. The user then inputs the departure airport and the arrival airport The computer may calculate the number of time zones to travel and the direction of travel. The process then displays the regimen to follow for each day in order to administer the proper amount of high intensity light for a desired period of time and at the right time. Suggestions may also be given regarding when to go to bed and when to wake up.

## Claims

1. A light therapy apparatus (10) comprising
a light emitting assembly (12) holding
a plurality of light emitting diodes (14) for emission of light and having a computer interface,
a presence detector (22) for detection of a person present in the field of emission of the light emitting assembly (12), and
a computer (20) that is interconnected with the light emitting assembly (12) through the computer interface for power supply of the light emitting assembly (12), and that is further adapted to
control emitted light intensity to be appropriate for light therapy,
whereby the user may simultaneously perform computer work during treatment, and to record the time that a person receives light treatment as detected by the presence detector (22).

2. A light therapy apparatus (10) according to claim 1, further comprising a second light emitting assembly (16) holding a plurality of light emitting diodes (18) for emission of light and having a second computer interface for connection with the computer (20) for control and power supply of the light emitting assembly (16).

3. A light therapy apparatus (10) according to any of the preceding claims, comprising light emitting diodes (14,18) with a capacity to emit blue light having a wavelength in the range from 435 nm to 500 nm.

4. A light therapy apparatus (10) according to any of the preceding claims, comprising multicoloured light emitting diodes (14, 18).

5. A light therapy apparatus (10) according to any of the preceding claims, comprising light sources for emission of white light.

6. A light therapy apparatus (10) according to any of the preceding claims, wherein the light emitting assembly (12) has a height less than 40 cm, such as less than 30 cm and a width less than 10 cm such as less than 5 cm, and depth less than 5 cm.

7. A light therapy apparatus (10) according to any of the preceding claims, wherein the light emitting assembly (12, 16) weighs less than 200 g.

8. A light therapy apparatus (10) according to any of the preceding claims, further adapted to automatically emit light when a person is present and to automatically stop light emission when the person leaves.

9. A light therapy apparatus (10) according to any of the preceding claims, wherein the light emitting assembly (12, 16) is integrated with a computer display.

10. A light therapy apparatus (10) according to any of the preceding claims, wherein the computer (20) is further adapted to control the light emitting assembly (12, 16) in such a way that the emitted light intensity as a function of time corresponds to the daylight as a function of time at a selectable position on the earth.

11. A light therapy apparatus (10) according to any of the preceding claims, wherein the computer (20) further comprises a jet lag calculator to be used by travellers to change sleep patterns and circadian rhythms.

## Patentansprüche

1. Lichttherapiegerät (10) umfassend
eine Lichtemissionsanordnung (12), welche eine Mehrheit von Lichtemissionsdioden (14) zur Emission von Licht aufnimmt und eine Computerschnittstelle aufweist,
einen Anwesenheitsmelder (22) zur Detektion einer in dem Emissionsfeld der Lichtemissionsanordnung (12) anwesenden Person, und
einen Computer (20), welcher über die Computerschnittstelle mit der Lichtemissionsanordnung (12) zur Stromversorgung der Lichtemissionsanordnung (12) verbunden ist und ferner zur Steuerung der Lichtintensität ausgebildet ist, damit diese für Lichttherapie geeignet ist, wobei der Benutzer während der Therapie gleichzeitig Computerarbeit ausführen kann, und zum Erfassen des Zeitraums der durch eine Person erhaltenen Lichttherapie, was durch den Anwesenheitsmelder (22) detektiert wird, ausgebildet ist.

2. Lichttherapiegerät (10) nach Anspruch 1, welches ferner eine zweite Lichtemissionsanordnung (16) umfasst, welche eine Mehrheit von Lichtemissionsdioden (18) zur Emission von Licht aufnimmt und eine zweite Computerschnittstelle zum Verbinden mit dem Computer (20) zur Steuerung und Stromversorgung der Lichtemissionsanordnung (16) aufweist.

3. Lichttherapiegerät (10) nach einem der vorgehenden Ansprüche, umfassend Lichtemissionsdioden (14, 18) mit einer Fähigkeit zur Emission von Blaulicht mit einer Wellenlänge im Bereich von 435 nm bis 500 nm.

4. Lichttherapiegerät (10) nach einem der vorgehenden Ansprüche, welches mehrfarbige Lichtemissionsdioden (14, 18) umfasst.

5. Lichttherapiegerät (10) nach einem der vorgehenden Ansprüche, welches Lichtquellen zur Emission von weißem Licht umfasst.

6. Lichttherapiegerät (10) nach einem der vorgehenden Ansprüche, wobei die Lichtemissionsanordnung (12) eine Höhe von weniger als 40 cm, wie beispielsweise weniger als 30 cm, und eine Breite von weniger als 10 cm, wie beispielsweise weniger als 5 cm, und eine Tiefe von weniger als 5 cm aufweist.

7. Lichttherapiegerät (10) nach einem der vorgehenden Ansprüche, wobei das Gewicht der Lichtemissionsanordnung (12, 16) weniger als 200 g beträgt.

8. Lichttherapiegerät (10) nach einem der vorgehenden Ansprüche, welches ferner dazu ausgebildet ist, selbsttätig Licht zu emittieren, wenn eine Person anwesend ist, und selbsttätig die Lichtemission zu arretieren, wenn die Person weggeht.

9. Lichttherapiegerät (10) nach einem der vorgehenden Ansprüche, wobei die Lichtemissionsanordnung (12, 16) mit einer Computeranzeige integriert ist.

10. Lichttherapiegerät (10) nach einem der vorgehenden Ansprüche, wobei der Computer ferner derart zur Steuerung der Lichtemissionsanordnung (12, 16) ausgebildet ist, dass die Intensität des emittierten Lichts als eine Funktion der Zeit dem Tageslicht als einer Funktion der Zeit an einer auswählbaren Position auf der Erde entspricht.

11. Lichttherapiegerät (10) nach einem der vorgehenden Ansprüche, wobei der Computer (20) ferner einen Jetlag-Rechner zur Verwendung durch Reisende zur Veränderung von Schlafmustern und Tagesrhythmen umfasst.

## Revendications

1. Appareil de photothérapie (10) comprenant
une assemblée émettant de la lumière (12), contenant
une pluralité de diodes électroluminescentes (14) pour l'émission de lumière et ayant une interface d'ordinateur,
un détecteur de présence (22) pour la détection d'une personne présente dans le domaine de l'émission de l'assemblée émettant de la lumière (12), et
un ordinateur (20) qui est interconnecté avec l'assemblée émettant de la lumière (12) par l'interface d'ordinateur pour l'alimentation en énergie de l'assemblée émettant de la lumière (12), et qui est en outre adapté
à vérifier si l'intensité lumineuse émise est appropriée pour la photothérapie, l'utilisateur étant capable de réaliser un travail assisté par ordinateur en même temps que le traitement, et
à enregistrer le temps pendant lequel une personne reçoit une photothérapie telle que détectée par le détecteur de présence (22).

2. Appareil de photothérapie (10) selon la revendication 1, comprenant en outre une deuxième assemblée émettant de la lumière (16) contenant une pluralité de diodes électroluminescentes (18) pour l'émission de lumière et présentant une deuxième interface d'ordinateur pour connexion avec l'ordinateur (20) pour la commande et l'alimentation en énergie de l'assemblée émettant de la lumière (16).

3. Appareil de photothérapie (10) selon l'une quelconque des revendications précédentes, comprenant des diodes électroluminescentes (14, 18) avec une capacité d'émettre la lumière bleue ayant une longueur d'onde comprise entre 435 nm et 500 nm.

4. Appareil de photothérapie (10) selon l'une quelconque des revendications précédentes, comprenant des diodes électroluminescentes multicolores (14, 18).

5. Appareil de photothérapie (10) selon l'une quelconque des revendications précédentes, comprenant les sources de lumière pour l'émission de la lumière blanche.

6. Appareil de photothérapie (10) selon l'une quelconque des revendications précédentes, dans lequel l'assemblée émettant de la lumière (12) présente une hauteur inférieure à 40 cm, p. ex. inférieure à 30 cm, et une largeur inférieure à 10 cm, p. ex. inférieure à 5 cm, et une profondeur inférieure à 5 cm.

7. Appareil de photothérapie (10) selon l'une quelconque des revendications précédentes, dans lequel l'assemblée émettant de la lumière (12, 16) pèse moins de 200 g.

8. Appareil de photothérapie (10) selon l'une quelconque des revendications précédentes, qui est en outre adapté à émettre de la lumière automatiquement quand une personne est présente et à arrêter l'émission de lumière automatiquement quand la personne quitte.

9. Appareil de photothérapie (10) selon l'une quelconque des revendications précédentes, dans lequel l'assemblée émettant de la lumière (12, 16) est intégrée avec un écran d'ordinateur.

10. Appareil de photothérapie (10) selon l'une quelconque des revendications précédentes, dans lequel l'ordinateur (20) est en outre adapté à commander l'assemblée émettant de la lumière (12, 16) de telle façon que l'intensité lumineuse émise en fonction du temps corresponde à la lumière du jour en fonction du temps à une position à sélectionner sur la terre.

11. Appareil de photothérapie (10) selon l'une quelconque des revendications précédentes, dans lequel l'ordinateur (20) comprend en outre un calculateur de décalage horaire (jet lag) à utiliser par les voyageurs pour changer leurs habitudes de sommeil et rythmes circadiens.
